Europäisches Patentamt

European Patent Office

Office européen des brevets

(11) Publication number: **0 245 782 B1**

# EUROPEAN PATENT SPECIFICATION

(45) Date of publication of patent specification: **19.08.92**

(51) Int. Cl.⁵: **A61M 1/34**, A61M 1/36, B01D 27/08

(21) Application number: **87106644.5**

(22) Date of filing: **07.05.87**

(54) **Device for filtering arterial blood.**

(30) Priority: **12.05.86 IT 2039486**

(43) Date of publication of application:
**19.11.87 Bulletin 87/47**

(45) Publication of the grant of the patent:
**19.08.92 Bulletin 92/34**

(84) Designated Contracting States:
**CH DE ES FR GB IT LI SE**

(56) References cited:
**GB-A- 1 501 665**
**US-A- 4 411 783**

(73) Proprietor: **DIDECO S.p.A.**
**Via Galilei 3**
**I-41037 Mirandola (Province of Modena)(IT)**

(72) Inventor: **Fini, Massimo**
**Via Savonarola 3**
**I-41037 Mirandola Provence of Modena(IT)**

(74) Representative: **Modiano, Guido et al**
**MODIANO, JOSIF, PISANTY & STAUB**
**Modiano & Associati Via Meravigli, 16**
**I-20123 Milano(IT)**

# Description

The invention relates to a device for filtering arterial blood.

It is known to provide extracorporeal blood circulation circuits, with arterial blood filtering devices which have the function of retaining microaggregates and microemboli, i.e. minute air bubbles occurring in blood, to prevent them from reaching the patient.

A very common type of these devices comprises a filter composed of three layers of net folded longitudinally and sunk, at the ends, in a supporting filler element, such as polyurethane resin, known as potting, this filter is arranged into a space inside a containment structure provided with connections for the inflow of the blood to be filtered and the outflow of the filtered blood, and with a connection for evacuating the air bubbles. Examples of these devices are shown in US-A-4 411 783 and in GB-A-1 501 665.

A disadvantageous characteristic of the known devices resides in the fact that the potting at the upper end of the filter, being in the form of a compact body, retains the air bubbles below which, blocked by the filter, return upwards in the spaces comprised between the folds of the net, thus preventing their evacuation.

The aim of the present invention is to provide a device for filtering arterial blood wherein any stagnation of air bubbles is completely prevented.

Within the scope of this aim, an object of the invention is to design a device inside which blood can circulate without being subject to dangers of haemolysis.

The aim proposed, as well as the object mentioned, are achieved by a device for filtering arterial blood, according to the invention, as defined in the characterizing part of claim 1.

Further advantages will become apparent from the description of a preferred, but not exclusive, embodiment of the invention, illustrated only by way of non-limitative example in the accompanying drawing, wherein:

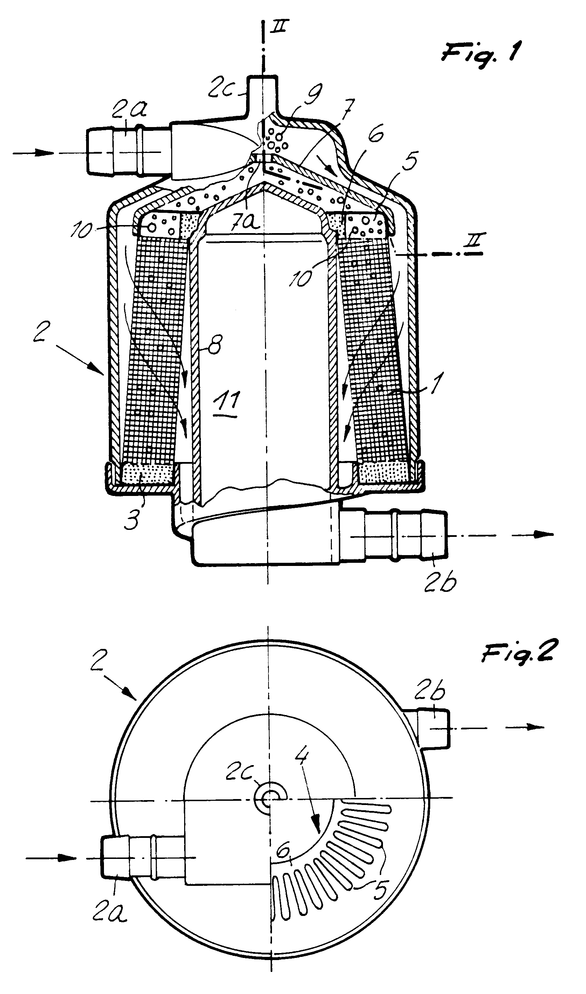

Fig. 1 is a cross section view of the invention along a plane passing through its axis;

Fig. 2 is a plan view of the invention with a partial cross section at the line II-II of figure 1.

With reference to the above described figures, the reference numeral 1 designates the filter composed, in a known manner, of three layers of net, two outer supporting layers and one intermediate filtering layer. The net is folded and lies in a portion of space having the shape of a hollow body of revolution inside a containment structure generally indicated by the reference numeral 2. The containment structure is provided with a blood inflow connection 2a, a blood outflow connection 2b, and with a connection 2c for the evacuation of the air bubbles contained in the blood.

The main peculiarity of the invention resides in the fact that, while the lower end of the filter 1 is sunk, in a known manner, in the compact annular block of supporting filler material 3 known as potting, consisting for example of polyurethane resin, the upper potting generally indicated by 4 comprises a plurality of tabs 5 deriving monolithically from a continuous inner band 6 and connecting the individual folds of the net.

The cover 7 with a central hole 7a covers the portion of space comprising the filter 1, which the elements 8 which derives from the bottom of the structure 2 acts as a support for said filter and as partial filler for the cavity 11 comprised therein, so as to reduce the amount of blood contained in the device.

The functional characteristics achieved with the device according to the invention are evident: blood enters through the tangential connection 2a and forms an eddy in the portion of space above the cover 7, which performs the double function of preventing the contact of the blood in this vorticose motion with the potting 4 which, due to its jagged shape, may create a harmful haemolysis effect, and of defining a region of blood at rest, lying thereunder after it has passed through the hole 7a, and therefore in a condition favourable for accepting the micro-bubbles trapped by the filter, as will be described hereinafter.

As an effect of the abovementioned vorticose motion, most of the air present separates from the blood in the form of bubbles 9, said bubbles being immediately evacuated through the connection 2c. The blood then descends, by following the path indicated by the arrows, in contact with the filter 1, then pass through the filter entering the portion of space adjacent to the element 8 and eventually flows out through the connection 2b; the air bubbles 10 still present in the blood are trapped by the filter 1 and go back up, without any obstacle, towards the underlying region of blood at rest below the cover 7, by virtue of the particular shape of the potting 4, from there they pass through the hole 7a into the upper region and then leave through the connection 2c.

Thus the described configuration of the potting at the upper end of the net is such as to completely prevent the stagnation of air bubbles inside the device, and it is important to observe that it has the maximum effectivness not only in devices of the type described, with the inflow of blood above and the outflow below, but in any type of devices regardless of their general structure, in particular it is effective also in devices having both the inflow and the outflow of the blood at the lower region.

The invention thus described is susceptible to numerous modifications and variations, all of which are within the scope of the claims.

## Claims

**1.** Device for filtering arterial blood, comprising a substantially cylindrical filter (1) composed of three layers of longitudinally folded net sunk at its ends in a supporting filler material (3,4) known as potting, said filter (1) being arranged inside a containment structure (2) provided with connections (2a,2b) for the inflow and the outflow of blood crossing said filter and a connection (2c) for evacuating air bubbles, said device further comprising a cover (7) for covering one of said ends sunk in potting (4) of said filter (1), characterized in that the potting (4) arranged at the cover (7) has the form of a substantially inner cylindrical band (6) from which a plurality of tabs (5) radially protrude, said cover has a center hole (7a) provided therein, the individual folds of said filter (1) being respectively sunk in said plurality of said tabs (5) and the cover (7) extending to cover the ends of said tabs (5), thereby passages being defined between said plurality of tabs (5) for the passage of gas bubbles (10) under the cover (7) and through said center hole (7a).

**2.** Device according to claim 1, characterized in that said filter (1) has a tapered configuration which converges towards the end arranged at the cover (7).

**3.** Device according to any of claims 1 or 2, characterized in that said containment structure (2) is of substantially cylindrical configuration having a cylindrical support element (8) arranged therein, said filter (1) being arranged between said containment structure (2) and said support element (8).

**4.** Device according to claim 3, characterized in that said cover (7) is arranged at an end of said containment structure (2) which is provided with the blood inflow connection (2a) and the air bubble evacuation connection (2c), said blood inflow connection (2a) being arranged above the cover (7) in a transverse circumferential direction with respect to the center axis of said containment structure (2) and said air bubble evacuation connection (2c) being arranged also above the cover (7) but in an axial direction at the center axis of said containment structure (2), said cover (7) being arranged inside said containment structure (2) so as to define a blood flow gap between said cover (7) and said containment structure (2), said blood outflow connection (2b) being arranged at the other end of the containment structure (2) also in a transverse circumferential direction with respect to the center axis of the containment structure (2).

## Patentansprüche

**1.** Vorrichtung zur Filterung von Arterienblut, enthaltend einen im wesentlichen zylindrischen Filter (1), bestehend aus drei Schichten aus längsgefaltenem Netz, die an ihren Enden in ein als Potting bekanntes Stützfüllmaterial (3, 4) getaucht sind, wobei der Filter (1) in einer Behälterstruktur (2) angeordnet ist, die mit Anschlüssen (2a, 2b) für den Zufluß und den Abfluß von den Filter durchquerendem Blut sowie mit einem Anschluß (2c) zum Abzug von Luftbläschen versehen ist, wobei die Vorrichtung weiterhin eine Abdeckung (7) enthält, die eines der in den Potting (4) des Filters (1) eingetauchten Enden abdeckt, dadurch gekennzeichnet, daß der Potting (4), der an der Abdeckung (7) angeordnet ist, die Form eines im wesentlichen inneren zylindrischen Gürtels (6) aufweist, von dem eine Anzahl von Vorsprüngen (5) in radialer Richtung hervorstehen, wobei die Abdeckung ein zentrales Loch (7a) aufweist, wobei die einzelnen Falten des Filters (1) jeweils in die genannten Vorsprünge (5) eingetaucht sind und sich die Abdeckung (7) zur über die Enden der Vorsprünge (5) erstreckt und diese abdeckt, wobei auf diese Weise Durchtrittsöffnungen zwischen den Vorsprüngen (5) gebildet werden, über die Gasbläschen (10) unter der Abdeckung (7) und durch das zentrale Loch (7a) durchtreten können.

**2.** Vorrichtung nach Anspruch 1, dadurch gekennzeichnet, daß der Filter (1) in Kegelform gestaltet ist, die sich nach dem an der Abdeckung (7) angeordneten Ende verjüngt.

**3.** Vorrichtung nach jedwedem der Ansprüche 1 oder 2, dadurch gekennzeichnet, daß die Behälterstruktur (2) im wesentlichen eine Zylinderform mit einem darin angeordneten zylindrischen Stützelement (8) aufweist, wobei der Filter (1) zwischen der Behälterstruktur (2) und dem Stützelement (8) angeordnet ist.

**4.** Vorrichtung nach Anspruch 3, dadurch gekennzeichnet, daß die Abdeckung (7) an einem Ende der Behälterstruktur (2) angeordnet ist, das mit dem Anschluß (2a) für den Zufluß von Blut sowie mit dem Anschluß (2c) zum Abzug von Luftbläschen versehen ist, wobei der Anschluß (2a) für den Zufluß von Blut oberhalb der Abdeckung (7) in gegensätzlicher Umfangsrichtung gegenüber der Zentralachse der Behälterstruktur (2) angeordnet ist und wobei der Anschluß (2c) zum Abzug von Luftbläschen gleichfalls oberhalb der Abdeckung (7), jedoch in axialer Richtung an der Zentralachse der Behälterstruktur (2) angeordnet ist, wobei die Abdeckung (7) innerhalb der Behälterstruktur (2) angeordnet ist, so daß eine Blutströmungsspalte zwischen der Abdeckung (7) und der Behälterstruktur (2) gebildet wird, wobei der Anschluß (2b) für den Abfluß von Blut am anderen Ende der Behälterstruktur (2) gleichfalls in gegensätzlicher Umfangsrichtung gegenüber der Zentralachse der Behälterstruktur (2) angeordnet ist.

## Revendications

1. Dispositif pour filtrer du sang artériel, comprenant un filtre sensiblement cylindrique (1) constitué de trois couches de tissu plié longitudinalement et plongé, à ses extrémités, dans un matériau de remplissage de support (3,4 ) formant ancrage, ledit filtre (1) étant agencé à l'intérieur d'une structure de confinement (2) munie de liaisons (2a,2b) pour l'entrée et la sortie du sang traversant ledit filtre, et d'une liaison (2c) pour évacuer les bulles d'air, ledit dispositif comprenant de plus un couvercle (7) pour recouvrir une desdites extrémités noyée dans l'ancrage (4) dudit filtre (1),
caractérisé en ce que l'ancrage (4) agencé au niveau du couvercle (7) présente la forme d'une bande interne sensiblement cylindrique (6) à partir de laquelle font saillie radialement une pluralité de pattes (5), ledit couvercle présente un trou central (7a) le traversant, les plis individuels dudit filtre (1) étant respectivement plongés dans ladite pluralité desdites pattes (5) et le couvercle (7) s'étendant pour recouvrir les extrémités desdites pattes (5), des passages étant ainsi définis entre ladite pluralité de pattes (5) pour le passage de bulles de gaz (10) sous le couvercle (7) et à travers ledit trou central (7a).

2. Dispositif selon la revendication 1,
caractérisé en ce que ledit filtre (1) présente une configuration conique qui converge vers l'extrémité prévue au niveau du couvercle (7).

3. Dispositif selon la revendication 1 ou 2,
caractérisé en ce que ladite structure de confinement (2) présente une configuration sensiblement cylindrique ayant un élément de support cylindrique (8) agencé dans celle-ci, ledit filtre (1) étant agencé entre ladite structure de confinement (2) et ledit élément de support (8).

4. Dispositif selon la revendication 3,
caractérisé en ce que ledit couvercle (7) est agencé à une extrémité de ladite structure de confinement (2) qui est munie de la liaison (2a) d'entrée du sang et de la liaison (2c) d'évacuation des bulles d'air, ladite liaison (2a) d'entrée du sang étant agencée au-dessus du couvercle (7) dans une direction circonférentielle transversale par rapport à l'axe central de ladite structure de confinement (2), et ladite liaison (2c) d'évacuation des bulles d'air étant agencée également au-dessus du couvercle (7) mais dans une direction axiale par rapport à l'axe central de ladite structure de confinement (2), ledit couvercle (7) étant agencé à l'intérieur de ladite structure de confinement (2) de façon à définir un espace d'écoulement du sang entre ledit couvercle (7) et ladite structure de confinement (2), ladite liaison (2b) de sortie du sang étant agencée à l'autre extrémité de la structure de confinement (2) également dans une direction circonférentielle transversale par rapport à l'axe central de la structure de confinement (2).

Fig. 1

Fig. 2